# EUROPEAN PATENT APPLICATION

(11) **EP 1 415 666 A1**
(43) Date of publication of application: **06.05.2004**
(21) Application number: 02745996.5
(22) Date of filing: 12.07.2002
(51) Int. Cl.: A61K 45/00, A61K 31/4045, A61P 27/02, A61P 27/06, C07D 209/42

(54) **MEDICINAL COMPOSITIONS FOR OPTHALMIC USE**

(30) Priority: 13.07.2001 JP 2001213624
(71) Applicant: Kissei Pharmaceutical Co., Ltd., Matsumoto-shi Nagano 399-8710 (JP)
(72) Inventor: YAMAZAKI, Kenji, Yasu-gun, Shiga 520-2422 (JP); HIROTSU, Ichiro, Takatsuki-shi, Osaka 56944-118 (JP); MIYATA, Hiroshi, Matsumoto-shi, Nagano 390-0316 (JP); KITAZAWA, Makio, Matsumoto-shi, Nagano 399-0011 (JP)
(74) Representative: HOFFMANN - EITLE
(86) International application number: PCT/JP2002/007088
(87) International publication number: WO 2003/006061

(57) **Abstract**

A pharmaceutical composition excellent for treatment or prevention of a glaucoma or an ocular hypertension, which inhibits the development of drug resistance, and which can be administered for a longer period of time is provided.

The invention relates to pharmaceutical compositions such as eye drops, which comprises an neutral antagonist of α_{1A}-AR as a effective ingredient.

## Description

### FILED OF THE INVENTION

This invention relates to a novel ophthalmic pharmaceutical composition. Specifically, the invention relates to an ophthalmic pharmaceutical composition which comprises at least one of α_{1A}-adrenergic receptor neutral antagonists as an effective ingredient.

### BACKGROUND ART

One of ophthalmologic diseases, glaucoma, is in general a disease in which visual performance is damaged by enhanced intraocular pressure, and includes a glaucoma caused by congenital anomaly, a glaucoma secondary to various ophthalmologic diseases and wounds, or surgery, and primary glaucoma wherein genetic factors or structural alterations affect the discharge canal of hydatoid from the angle. Although glaucoma occurs at any age from the infancy to the elderly, it frequently develops after middle age, and leads to blindness. Thus, this adult disease should be necessary to be prevented and early treated.

Glaucoma that would be caused by various factors is usually treated or prevented by use of drugs having an activity for reducing intraocular pressure. Drug treatment of glaucoma generally continues over a longer period of time, which arises serious problems on side effects or drug resistance. Specifically, glaucoma has been previously treated or prevented by use of parasympathomimetic drugs, sympathomimetic drugs, prostaglandin derivatives, and β-blockers, all of which produce some side effects, and therefore new type of drugs has been desired. Under the circumstance, α₁-adrenergic receptor (hereinafter, referred to as α₁-AR) antagonists have been developed, which have an activity for enhancing outflow of hydatoid that plays an important role in nutrient supply and metabolism in corneas and ocular lens.

Previous pharmacological studies on α₁-AR, and the cloning of the gene of the receptor accelerated the finding of the three types of subtype, α_{1A}-adrenergic receptor subtype (hereinafter, referred to as α_{1A}-AR), α_{1B}-adrenergic receptor subtype (hereinafter, referred to as α_{1B}-AR), and α_{1D}-adrenergic receptor subtype, and the wide examinations on the localization and the functions of these subtypes in various animals and at diverse organs in human. For example, there have been reported that α_{1A}-AR predominately exists in rabbit irides (British Journal of Pharmacology, Vol. 127, No.6, pp.1367-1374 (1999)), and that the mRNA of α_{1A}-AR was detected in the human retina, showing that α_{1A}-AR exists in human retinae (Exp. Eye Res., Vol.70, pp.51-60 (2000)).

Recently, there have been reported that G protein-coupled receptors such as α-adrenergic receptors, as well as β-adrenergic receptors and histamine receptors balance certain equilibrium conditions between an inactive form and an active form, and that only active forms induce physiological responses via intracellular signal transduction system involving protein kinase C (Pharmacol. Rev., Vol. 48, pp.413-463(1996); Trends Pharmacol. Sci., Vol. 16, pp.89-97(1995)). Further, actions of agonists and antagonists of those receptors have been investigated, and it has been reported that a large member of typical antagonists are inverse agonists that shift the equilibrium conditions of the receptors between an inactive and active forms to the inactive form, and a continued administration of an antagonist that is higher in capability to shift the equilibrium conditions to the inactive form (hereinafter, referred to as inverse agonist activity) results in increased number of the receptor in compensation for tentative inhibition of intracellular signal transduction system (Trends in Pharmacological Sciences, Vol.16, pp. 10-13 (1995); Trends in Pharmacological Sciences, Vol. 18, pp.468-474 (1997); Trends in Biochemical Sciences, Vol.23, pp.418-422 (1998) and the like). For example, H₂ -histamine receptor antagonists, cimetidine and ranitidine, which are known as drugs for treatment of gastric and duodenal ulcer diseases, are higher in inverse agonist activity, and therefore, continued administration of those H₂ -histamine receptor antagonists will induce the increase in the number of H₂ -histamine receptor, and will in turn cause development of resistance (attenuated activities) and rebound phenomenon such as improved secretion of gastric acid after the discontinuation, which have been acknowledged as a problem. In addition to H₂ -histamine receptor, β₂-adrenergic receptors and α_{1B}-AR have been actively examined using variants thereof, and also various antagonists have been investigated for inverse agonist activities (Proc. Natl. Acad. Sci. USA., Vol.93, pp.6802-6807 (1996); Biochem. J., Vol. 325, pp.733-739 (1997)).

Glaucoma requires a continued control of intraocular pressure. Accordingly, when a typical antagonist that is higher in inverse agonist activity is continuously administered for treatment of glaucoma, it has been acknowledged as problems that drug resistance that attenuates an antagonist activity, and rebound phenomenon that deteriorates the condition after the discontinuation are likely developed, that increased dose due to drug resistance makes side effects worse, and that the most or the complete efficacy of the antagonist is lost. For example, it has been reported that, although corynanthine (methyl 17α-hydroxyyohimban-16β-carboxylate) was demonstrated to have an activity for reducing intraocular pressure at concentrations of 2 to 5% in eye drops in the clinical trial of patients suffering from ocular hypertension that is associated with aberrant intraocular pressure in average over the defined value, the continued administration thereof for a week developed drug resistance (Ophthalmology, Vol. 92, No. 7, pp.977-980 (1985)). Further, an additional α₁-AR antagonist, bunazosin hydrochloride (1-(4-amino-6,7-dimethoxy-2-quinazolinyl)-4-butyrylhexahydro-1H-1,4-diazepin hydrochloride) was known to hardly develop drug resistance, but has been reported to initiate the attenuation in an activity to reduce intraocular pressure after 4-weeks continuous administration of dose of 0.05 % eye drop in the clinical trial of patients suffering from primary closed angle glaucoma (among primary glaucoma, chronically progressed one) and ocular hypertension, thus the 0.05 % eye drop being less effective than 0.01 % eye drop (Journal of the eye, Vol. 11, No. 3, pp.423-429 (1994); Journal of the eye, Vol. 11, No. 4, pp.631-635 (1994)).

As described above, the development of drug resistance by use of drugs for reducing intraocular pressure has been important issue in the treatment of ophthalmologic diseases, especially glaucoma and ocular hypertensions in view of the fact that such treatment would be continued for a long period of time. It is believed that the incidence rate of drug resistance would be increased if dose to be administered is increased according to the attenuation of activity to reduce intraocular pressure due to the continuous administration of drugs.

### DISCLOSURE OF THE INVENTION

The present invention aims at providing excellent pharmaceutical compositions effective in treatment or prevention of ophthalmologic diseases, especially glaucoma and ocular hypertensions, which are capable of inhibiting the development of drug resistance due to continuous administration, of being administered continuously for a long period of time, and of being administered to a wide rage of patients.

The inventors of the present application examined various α₁-AR antagonists in order to obtain therapeutic agents effective in treatment of glaucoma that do not develop any drug resistance, and found antagonists that exhibit no or little inverse agonist activity for α_{1A}-AR. The present invention is based on this finding.

Thus, the invention relates to
(1) An ophthalmic pharmaceutical composition which comprises an α_{1A}-adrenergic receptor neutral antagonist as an effective ingredient, preferably, the pharmaceutical composition according to the present invention wherein the α_{1A}-adrenergic receptor neutral antagonist is selected from the group consisting of a compound of formula: 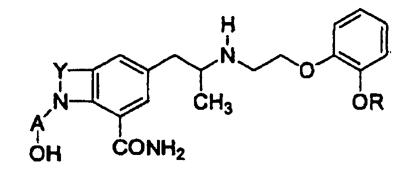 in which A is a lower alkylene, R is a lower alkyl or a halo(lower alkyl), and Y is ethylene or vinylene; a prodrug thereof, and a pharmaceutically acceptable salt of them; more preferably the α_{1A}-adrenergic receptor neutral antagonist is selected from the group consisting of (R)-5-[2-[[2-(2-ethoxyphenoxy)ethyl]amino]propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]propyl]-1H-indole-7-carboxamide, a prodrug thereof, and a pharmaceutically acceptable salt of them; even more preferably the prodrug is (R)-3-[7-carbamoyl-5-[2-[[2-(2-ethoxyphenoxy)ethyl]amino]propyl]-1H-indol-1-yl]propyl pivalate, or a pharmaceutically acceptable salt thereof; or
   the pharmaceutical composition according to the present invention, which is used together with an α_{1A}-adrenergic receptor inverse agonist, preferably the pharmaceutical composition, which comprises further an α_{1A}-adrenergic receptor inverse agonist; or
   the pharmaceutical composition according to the present invention, which is used for treatment or prevention of a glaucoma or an ocular hypertension;
(2) A pharmaceutical composition as used for α_{1A}-adrenergic receptor neutral antagonist, which comprises at least one selected from the group consisting of (R)-5-[2-[[2-(2-ethoxyphenoxy)ethyl]amino]-propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]-propyl]-1H-indole-7-carboxamide, a prodrug thereof, and a pharmaceutically acceptable salt of them; preferably a pharmaceutical composition as used for α_{1A}-adrenergic receptor neutral antagonist according to the present invention, which comprises (R)-3-[7-carbamoyl-5-[2-[[2-(2-ethoxyphenoxy)ethyl]amino]propyl]-1H-indol-1-yl]propyl pivalate, or a pharmaceutically acceptable salt thereof;
(3) A pharmaceutical composition comprising an α_{1A}-adrenergic receptor neutral antagonist, which is for prevention of a side effect by an α_{1A}-adrenergic receptor inverse agonist; preferably the pharmaceutical composition according to the present invention, which comprises further an α_{1A}-adrenergic receptor inverse agonist;
(4) A method for treating or preventing a glaucoma or an ocular hypertension, which comprises administering a therapeutically effective amount of an α_{1A}-adrenergic receptor neutral antagonist to a patient in need; preferably the method according to the present invention, wherein the α_{1A}-adrenergic receptor neutral antagonist is selected from the group consisting of a compound of formula: 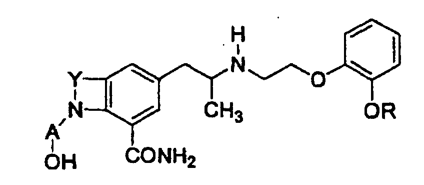 in which A is a lower alkylene, R is a lower alkyl or a halo(lower alkyl), and Y is ethylene or vinylene; preferably (R)-5-[2-[[2-(2-ethoxyphenoxy)-ethyl]amino]propyl]-1-(3-hydroxypropyl)-1H-indole-7-carboxamide, (R)-1-(3-hydroxypropyl)-5-[2-[[2-(2-(2,2,2-trifluoroethoxy)phenoxy]ethyl]-amino]propyl]-1H-indole-7-carboxamide, and a prodrug thereof, more preferably (R)-3-[7-carbamoyl-5-[2-[[2-(2-ethoxyphenoxy)ethyl]amino]-propyl]-1H-indol-1-yl]propyl pivalate, and a pharmaceutically acceptable salt thereof; or
   the method according to the present invention, which comprises administering a therapeutically effective amount of an α_{1A}-adrenergic receptor neutral antagonist to a patient who are receiving an α_{1A}-adrenergic receptor inverse agonist;
(5) A method for preventing a side effect occurred in a patient who is receiving an α_{1A}-adrenergic receptor inverse agonist, which comprises administering a therapeutically effective amount of an α_{1A}-adrenergic receptor neutral antagonist to the patient; and
(6) Use of an α_{1A}-adrenergic receptor neutral antagonist for the manufacture of a pharmaceutical composition for treatment or prevention of a glaucoma or an ocular hypertension; preferably the use according to the present invention, wherein the α_{1A}-adrenergic receptor neutral antagonist is selected from the group consisting of a compound of formula: 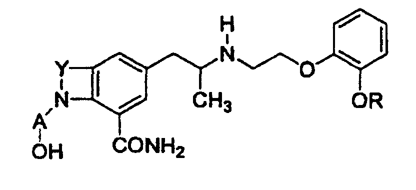
in which A is a lower alkylene, R is a lower alkyl or a halo(lower alkyl), and Y is ethylene or vinylene; preferably (R)-5-[2-[[2-(2-ethoxyphenoxy)-ethyl]amino]propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]-amino]propyl]-1H-indole-7-carboxamide, and a prodrug thereof, more preferably (R)-3-[7-carbamoyl-5-[2-[[2-(2-ethoxyphenoxy)ethyl]amino]-propyl]-1H-indol-1-yl]propyl pivalate, and a pharmaceutically acceptable salt thereof; or

Use of an α_{1A}-adrenergic receptor neutral antagonist and an α_{1A}-adrenergic receptor inverse agonist, for the manufacture of a pharmaceutical composition for treatment or prevention of a glaucoma or an ocular hypertension.

### BRIEF DESCRIPTION OF DRAWINGS

Figure 1 depicts a graph that shows intracellular levels of IP₃ produced in CHO cells expressing an α_{1A}-AR (variant and wild). The vertical axis represents intracellular amounts of IP₃ (pmol/ 10⁶ cells).
Figure 2 depicts a graph showing effects of test drugs on expression level of the α_{1A}-AR in CHO cells expressing the variant receptor. The vertical axis represents expression level of the α_{1A}-AR (fmol/mg protein), and the horizontal axis shows the presence or absence of the test drug and the kinds of the same.

### BEST MODE FOR CARRYING OUT THE INVENTION

### (1) Pharmaceutical compositions which comprise an α_{1A}-adrenergic receptor neutral antagonist as an effective ingredient

In the first embodiment, the present invention provides an ophthalmic pharmaceutical composition which comprises an α_{1A}-adrenergic receptor neutral antagonist as an effective ingredient, specifically a pharmaceutical composition as such for treatment or prevention of a glaucoma or an ocular hypertension.

As used herein, the term "α_{1A}-AR neutral antagonist" or "α_{1A}-adrenergic receptor neutral antagonist" means an antagonist of α_{1A}-AR that induces no increase in the number of receptor even after continuous administration, or that causes neither drug resistance that attenuates an antagonist activity, nor rebound phenomenon that deteriorates the condition after the discontinuation.

Contrary to conventional antagonists, many of which are inverse agonists that shift the equilibrium conditions of receptors between an inactive and active forms to the inactive form, neutral antagonists never affect the equilibrium conditions of receptors between an inactive and active forms. Although continued administration of an antagonist that is higher in inverse agonist activity likely develops drug resistance and rebound phenomenon that deteriorates the condition after the discontinuation, neutral antagonists are not believed to cause such disadvantage as associated with the continued administration of inverse agonists. Neutral antagonists were mentioned in Pharmacia ,Vol.33, No.6 pp.617-621 (1997), but any specific details including the mechanism of neutral antagonists have not been discussed.

The present inventors used the CHO cells that express the variant α_{1A}-AR wherein the active form is predominant so as to examine various indole derivatives for their capability to increase the number of the α_{1A}-AR. As a result, the inventors found that (R)-5-[2-[[2-(2-ethoxyphenoxy)ethyl]amino]propyl]-1-(3-hydroxypropyl)-1H-indole-7-carboxamide hydrochloride (hereinafter, referred to as KRG-3333) that surely exhibits an α_{1A}-AR antagonist activity induced no increase in the α_{1A}-AR number. This finding shows that KRG-3333 is an α_{1A}-AR neutral antagonist for the first time.

"Alfa_{1A}-AR neutral antagonist" as used herein also encompasses antagonists of α_{1A}-AR, which exhibit a significantly low inverse agonist activity to induce a little or almost no increase in the number of α_{1A}-AR when determined by the determination method for inverse agonist activity as described herein, or a similar method thereto, in other words, those which exhibit a little or substantially no inverse agonist activity.

Determination of inverse agonist activity may be conducted by the method using variant α_{1A}-AR as described in Example 1 hereinafter, or a method similar to the same. In brief, inverse agonist activity may be detected by the produced amount of inositol-1,4,5-trisphosphate (hereinafter, referred to as IP₃) or increased number of receptor. IP₃ is one of signal transduction messengers that are produced in the course of activation of protein kinase C.

More specifically, an α_{1A}-AR neutral antagonist may be screened by culturing the CHO cells that express the variant α_{1A}-AR wherein the active form is predominant in the presence of a candidate for the antagonist, and then determining change in the number of receptor. CHO cells that express the variant α_{1A}-AR wherein the active form is predominant may be prepared according to the method as described in the publication (British Journal of Pharmacology, Vol. 127, pp.962-968 (1999); British Journal of Pharmacology, Vol.131, pp.546-552 (2000)). In the present screening, the determination whether or not a candidate is an α_{1A}-AR neutral antagonist may be conducted for example by parallel screening the candidate and an inverse agonist, Bunazosin hydrochloride under the same conditions, and comparing the change in the number of α_{1A}-AR. According to the present invention, "α_{1A}-AR neutral antagonist" as an effective ingredient in the present composition should show about 1/3 or less, preferably about 1/6 or less, more preferably substantial zero, when the increased number of α_{1A}-AR in the presence of Bunazosin hydrochloride that is an inverse agonist is assumed as one.

The method as described above corresponds to a process of screening for an agent that is used in the ophthalmologic field, specifically for treatment or prevention of a glaucoma or an ocular hypertension, and that develop substantially no drug resistance, which comprises culturing cells that express variant α_{1A}-adrenergic receptor wherein the active form is predominant in the presence of a candidate for an α_{1A}-adrenergic receptor neutral antagonist, and then determining whether or not the number of receptor is changed; preferably the process further comprising comparing with the increased number of the receptor in the presence of ophthalmic inverse agonists such as Bunazosin hydrochloride. Such processes are also fallen within the scope of the present invention.

Any α_{1A}-AR neutral antagonist that is obtained by the screening as described above, and that is ophthalmologically acceptable may be comprised as an effective ingredient in the composition of the present invention. It will be readily for those skilled in the art to screen various candidates for α_{1A}-AR antagonist, and obtain ophthalmologically acceptable α_{1A}-AR neutral antagonists that are also suitable for formulation.

As used herein, α_{1A}-AR neutral antagonists include a compound of formula (I): 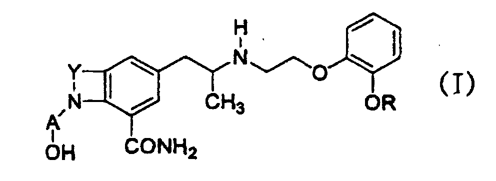 in which A is a lower alkylene, R is a lower alkyl or a halo(lower alkyl), and Y is ethylene or vinylene; a prodrug thereof, and a pharmaceutically acceptable salt of them. In formula (I), "lower alkylene" of substituent A means a C₂-C₆ straight or branched alkylene group, and includes ethylene, trimethylene, propylene, tetramethylene, pentamethylene and hexamethylene, provided that a bonding position of the hydroxy group bound to substituent A is other than α position. "Alkyl" of substituent R means a C₁-C₆ straight or branched alkyl group, and includes methyl, ethyl, propyl, isopropyl, butyl, isobutyl, sec-buthyl, pentyl, isopentyl, neopentyl, 1-methylbutyl, 2-methylbutyl, and hexyl. "Halo(lower alkyl)" means the above "alkyl" substituted with one to three same or different halogens selected from the group consisting of fluorine, chlorine, bromine and iodine, and includes, for example, trifluoromethyl, and trifluoroethyl.

Preferred α_{1A}-AR neutral antagonists as used herein are (R)-5-[2-[[2-(2-ethoxyphenoxy)ethyl]amino]propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]propyl]-1H-indole-7-carboxamide, or a pharmaceutically acceptable salt thereof.

Alpha_{1A}-AR neutral antagonists comprised in the pharmaceutical compositions of the present invention may be used as they are in a form of active compound, or in a form of a prodrug that is converted into an active compound during or after cornea transmission to exert an α_{1A}-AR antagonist activity.

Prodrugs as used herein include compounds of formula (I) of which the hydroxy or the imino group are appropriately introduced with a group which can be used to provide prodrugs, and such group as providing prodrugs are as described in Pharmaceutical Research and Development, Vol. 7 Molecular design, pp163-198, Publisher, Tokyo Hirokawa Publisher Company; Drugs of the Future, 16(5), 443-458 (1991); Drug Bioavailability Scientific Estimation and Improvement, pp. 133-153, Publisher, Gendaiiryosha Co., Ltd. Pharmaceutically acceptable salts include acid addition salts formed with mineral acids such as hydrochloric acid, hydrobromic acid, hydriodic acid, sulfuric acid, nitric acid, and phosphoric acid; and acid addition salts formed with organic acids such as carbonic acid, formic acid, acetic acid, propionic acid, butyric acid, oxalic acid, citric acid, succinic acid, tararic acid salts, fumaric acid, malonic acid, maleic acid, malic acid, lactic acid, adipic acid, benzoic acid, salicylic acid, methanesulfonic acid, p-toluenesulfonic acid, glutamic acid, and aspartic acid.

Preferred prodrug is (R)-3-[7-carbamoyl-5-[2-[[2-(2-ethoxyphenoxy)ethyl]amino]propyl]-1H-indol-1-yl]propyl pivalate or a pharmaceutically acceptable salt thereof (hereinafter, the hydrochloride is referred to as KRG-3332).

The indole derivatives as described above may be prepared by well known method in the art, or commercially available. For example, the compound of formula (I), a prodrug thereof, and a pharmaceutically acceptable salt of them may be prepared according to the methods as described in Japanese Patent Publication (Kokai) No. 330725/1995; Japanese Patent Publication (Kokai) No. 330726/1995; WO99/43652, or a similar method thereto. In brief, those compounds may be prepared by the following steps including treating the relevant indoline carbonitrile derivatives with aqueous sodium hydroxide and hydrogen peroxide liquid, protecting the secondary nitrogen atom of the resultant carboxamide with a protecting group such as *tert*-butoxycarbonyl according to conventional method, if desired, introducing it with a protecting group by use of prodrug-producing reagents such as halide pivalate and converting the same to ester according to conventional method, then oxidizing the indoline ring in the presence of metal catalysts such as palladium-carbon, and ammonium formate, and then removing the protecting group from the nitrogen atom.

Alpha_{1A}-AR neutral antagonists comprised in the pharmaceutical compositions of the present invention may be used as pharmaceutically acceptable salts. The Pharmaceutically acceptable salts include acid addition salts formed with mineral acids such as hydrochloric acid, hydrobromic acid, hydriodic acid, sulfuric acid, nitric acid, and phosphoric acid; and acid addition salts formed with organic acids such as carbonic acid, formic acid, acetic acid, propionic acid, butyric acid, oxalic acid, citric acid, succinic acid, tararic acid salts, fumaric acid, malonic acid, maleic acid, malic acid, lactic acid, adipic acid, benzoic acid, salicylic acid, methanesulfonic acid, p-toluenesulfonic acid, glutamic acid, and aspartic acid; as well as salts formed with organic amines such as 2-aminoethanol, piperidine, morpholine, and pyrrolidine, and salts formed with inorganic bases such as sodium, potassium, calcium and magnesium. Also, compounds comprised in the pharmaceutical compositions of the present invention include hydrates of the compounds or the salts as mentioned above and solvates of the same formed with a pharmaceutically acceptable solvent such as ethanol.

As used herein, ophthalmic pharmaceutical compositions means pharmaceutical compositions that may treat an ophthalmologic disease. Glaucoma, a disease to be treated in the present invention encompasses various diseases in which visual performance is damaged by enhanced intraocular pressure, irrespective of the causes such as congenital anomaly, wounds and surgery. Glaucoma usually renders average intraocular pressure exceed 21mmHg, but additionally includes normal tension glaucoma wherein a glaucomatous alteration is found in visual field and optic papilla even when average intraocular pressure is 21mmHg or less, which glaucoma is also fallen within the meaning of the glaucoma as used herein.

"Ocular hypertensions", which is also directed to the present pharmaceutical composition is diseases associated with aberrant intraocular pressure wherein average intraocular pressure exceeds 21mmHg. The diseases as used herein include an ocular hypertension without aberration in visual field and optic papilla (high ocular tension), which could advance to glaucoma, and therefore early treatment of such disease is important in view of prevention of glaucoma. In this context, pharmaceutical compositions for treatment of ocular hypertensions are also fallen within the scope of the present invention.

Preferred pharmaceutical compositions according to the present invention are ophthalmic pharmaceutical compositions comprising a compound of formula (I), a prodrug thereof, or a pharmaceutically acceptable salt of them, which induce no increase in the number of receptor even after continuous administration, or which causes neither drug resistance that attenuates an antagonist activity nor rebound phenomenon that deteriorates the condition after the discontinuation.

When used for actual treatment or prevention, effective ingredient comprised in the pharmaceutical composition of the invention may be used as pharmaceutical compositions in various forms depending on the usage suitable for treatment of ophthalmologic diseases such as eye drop, eye ointment, injection, oral medicaments such as tablet. Topical administration as eye drop is the most typical. The eye drops and the other pharmaceutical compositions may be prepared according to conventional pharmaceutical technique. For example, eye drops among ophthalmic formulations may be prepared by the following steps including adding the effective ingredient of the invention to a sterile purified water, if necessary supplementing with an appropriate solubilizing agent or suspending agent, then dissolving or suspending the ingredient therein, if necessary supplementing further with a preservative, an agent making isotonic, or a pH modifier, and removing dusts and sterilizing the solution or the suspension.

Although a dose of an effective ingredient, α_{1A}-AR neutral antagonist, varies depending on particular kind of the ingredient, sex, age, body weight, conditions of the patient when the pharmaceutical composition of the invention is used for actual treatment or prevention, for example in the case of eye drop comprising KRG-3332 as an effective ingredient, the eye drop having a concentration of about 0.001 to 0.5%, preferably about 0.005 to 0.1%, more preferably about 0.01 to 0.1% is administered to the eye once to several times per day.

In this embodiment, the present invention encompasses a method for treating or preventing a glaucoma or an ocular hypertension, which comprises administering a therapeutically effective amount of an α_{1A}-adrenergic receptor neutral antagonist to a patient in need. The α_{1A}-adrenergic receptor neutral antagonist as used therein are as defined above.

Additionally, in this embodiment, the present invention encompasses use of an α_{1A}-adrenergic receptor neutral antagonist for the manufacture of a pharmaceutical composition for treatment or prevention of a glaucoma or an ocular hypertension. The α_{1A}-adrenergic receptor neutral antagonist as used for the manufacture of the pharmaceutical composition are as defined above.

As another aspect, the present invention also encompasses a pharmaceutical composition according to the present invention, which is used together with an α_{1A}-AR inverse agonist, specifically an antagonist that is higher in inverse agonist activity.

According to the present aspect, types of usage of the pharmaceutical composition "which is used together with an α_{1A}-AR inverse agonist" include the type of usage of the pharmaceutical composition which comprises both an α_{1A}-AR inverse agonist and an α_{1A}-AR neutral antagonist, and the type of concomitant usage of independent pharmaceutical compositions comprising each ingredient. Preferably, the pharmaceutical composition which comprises both an α_{1A}-AR inverse agonist and an α_{1A}-AR neutral antagonist is used.

As described above, "α_{1A}-AR inverse agonist" as used herein means a agent that attenuates an antagonist activity due to continuous administration, and that likely causes drug resistance and rebound phenomenon that deteriorates the condition after the discontinuation. When the pharmaceutical composition of the present invention and an inverse agonist are concomitantly administered, such disadvantages due to continuous administration hardly occur, and dose of α_{1A}-AR inverse agonist can be lowered, thereby developing little side effects as mentioned above. Consequently, it is possible to obtain a formulation that not only improve a therapeutic effect, but also maintain the efficacy for a longer period of time, thus providing limited side effect and high safety. Alfa_{1A}-AR inverse agonist as used herein are not limited to particular species as long as it is ophthalmologically acceptable. Examples of α_{1A}-AR inverse agonist include bunazosin hydrochloride (1-(4-amino-6,7-dimethoxy-2-quinazolinyl)-4-butyrylhexahydro-1H-1,4-diazepin hydrochloride), terazosin hydrochloride (1-(4-amino-6,7-dimethoxy-2-quinazolinyl)-4-(tetrahydro-2-furoyl)piperazine hydrochloride dihydrate), doxazosin mesilate (1-(4-amino-6,7-dimethoxy-2-quinazolinyl)-4-(1,4-benzodioxan-2-yl carbonyl)piperazine methansulfonate), and urapidil (6-[[3-[4-(o-methoxyphenyl)-1-piperazinyl]propyl]amino]-1,3-dimethyl uracil, and bunazosin hydrochloride is preferred. Those drugs are commercially available, and some compounds may be prepared according to the method as described in the following, or a similar method thereto:
WO94/05628, Japanese Patent Publication (Kokai) No. 16417/1987, Japanese Patent Publication (Kokai) No. 103177/1981, US Patent No. 5919931, Japanese Patent Publication (Kokai) No. 27588/1979, Japanese Patent Publication (Kokai) No. 48678/1977, Japanese Patent Publication (Kokai) No. 98792/1979, US Patent No. 3957786, and US Patent No. 4067982.

When the pharmaceutical composition of the present invention comprising an α_{1A}-AR neutral antagonist and an inverse agonist are concomitantly administered, each composition principally comprises an appropriate amount of each antagonist. Alternatively, when the pharmaceutical composition of the present invention comprising an α_{1A}-AR neutral antagonist and an inverse agonist are separately prepared, each composition is formulated according to conventional method, and the dose may be adjusted as appropriate when administrated. Such formulation and preparation are well known in the art.

In the context of the present embodiment, the invention encompasses a method for treating or preventing a glaucoma or an ocular hypertension, which comprises administering a therapeutically effective amount of an α_{1A}-adrenergic receptor neutral antagonist to a patient who are receiving an α_{1A}-adrenergic receptor inverse agonist.

Additionally, in the context of the present embodiment, the invention encompasses use of an α_{1A}-adrenergic receptor neutral antagonist and an α_{1A}-adrenergic receptor inverse agonist, for the manufacture of a pharmaceutical composition for treatment or prevention of a glaucoma or an ocular hypertension.

### (2) Pharmaceutical compositions as used for α_{1A}-adrenergic receptor neutral antagonist, which comprises an indole derivative as defined above

In the second embodiment, the present invention provides a pharmaceutical composition as used for α_{1A}-adrenergic receptor neutral antagonist, which comprises at least one selected from the group consisting of a compound of formula (I), a prodrug thereof, and a pharmaceutically acceptable salt of them; preferably a pharmaceutical composition as used for α_{1A}-adrenergic receptor neutral antagonist, which comprises at least one selected from the group consisting of (R)-5-[2-[[2-(2-ethoxyphenoxy)ethyl]amino]-propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]-propyl]-1H-indole-7-carboxamide, a prodrug thereof, and a pharmaceutically acceptable salt of them; more preferably a pharmaceutical composition as used for α_{1A}-adrenergic receptor neutral antagonist, which comprises (R)-3-[7-carbamoyl-5-[2-[[2-(2-ethoxyphenoxy)ethyl]amino]propyl]-1H-indol-1-yl]propyl pivalate, or a pharmaceutically acceptable salt thereof. The effective ingredient in those pharmaceutical compositions are described in WO99/43652, but those compounds have been found to exhibit an α_{1A}-AR neutral antagonist activity according to the present invention for the first time.

In the present embodiment, the pharmaceutical compositions as used for α_{1A}-adrenergic receptor neutral antagonist include pharmaceutical compositions for treatment or prevention of diseases desired to be effected by neutral antagonists, such as a glaucoma, an ocular hypertension, distress in urination, arrhythmia, cardiac enlargement, erectile dysfunction, sympathetic pains, hyperlipemia, and diabetes mellitus, all of which are subjected to continuous administration of the drugs.

### (3) Pharmaceutical compositions, which is for prevention of a side effect by an α_{1A}-adrenergic receptor inverse agonist

In the third embodiment, the present invention provides a pharmaceutical composition comprising an α_{1A}-adrenergic receptor neutral antagonist, which is for prevention of a side effect by an α_{1A}-adrenergic receptor inverse agonist. "Side effects by an α_{1A}-adrenergic receptor inverse agonist" mean drug resistance that attenuates an antagonist activity due to continuous administration, and rebound phenomenon that deteriorates the condition after the discontinuation as described above.

The present inventors used the CHO cells that express the variant α_{1A}-AR wherein the active form is predominant to conduct an experiment wherein an α_{1A}-AR inverse agonist is used together with KRG-3333, and surprisingly found that KRG-3333 drastically decreased the inverse agonist activity. Accordingly, the invention prevents the side effects due to continuous administration of an α_{1A}-AR inverse agonist, and makes it possible to lower the does of an α_{1A}-AR inverse agonist, the latter being also helpful to prevent the side effects. In other words, the present embodiment of the invention relates to a pharmaceutical composition for prevention of a side effect by an α_{1A}-adrenergic receptor inverse agonist, which comprises an α_{1A}-adrenergic receptor neutral antagonist. As used herein, the α_{1A}-AR inverse agonist is as defined above.

One example of the present embodiment include a pharmaceutical composition comprising not only an α_{1A}-AR neutral antagonist but also an α_{1A}-AR inverse agonist.

In the context of the embodiment, the invention encompasses a method for preventing a side effect occurred in a patient who is receiving an α_{1A}-adrenergic receptor inverse agonist, which comprises administering a therapeutically effective amount of an α_{1A}-adrenergic receptor neutral antagonist to the patient;

The following reference examples, examples, and formulations are presented for the purpose of further illustration of the invention, and those are not intended to limit the invention in any respect.

### EXAMPLES

### Reference Example 1

### Preparation of (R)-3-[7-carbamoyl-5-[2-[[2-(2-ethoxyphenoxy)ethyl]-amino]propyl]-1H-indol-1-yl]propyl pivalate

To 120 mL of a solution of potassium carbonate (32.3 g) in a distilled water that had been added with 120 mL of ethyl acetate, 12.0 g of (R)-3-[5-(2-aminopropyl)-7-cyano-2,3-dihydro-1H-indol-1-yl]propyl benzoate L-tartrate (as prepared according to the publication as mentioned above) was added in small portions, and the mixture was stirred for an hour. The reaction mixture was added with ethyl acetate to conduct the extraction, and the ethyl acetate phase was washed with a 10% aqueous potassium carbonate and with a saturated brine, and dried over magnesium sulfate. The solvent was evaporated *in vacuo* to give 8.98 g of (R)-3-[5-(2-aminopropyl)-7-cyano-2,3-dihydro-1H-indol-1-yl]propyl benzoate as a brown oil.

To a solution of 8.98 g of the resultant compound, (R)-3-[5-(2-aminopropyl)-7-cyano-2,3-dihydro-1H-indol-1-yl]propyl benzoate as dissolved in 43 mL of *tert*-butanol, 7.02 g of 2-(2-ethoxyphenoxy)ethyl methansulfonate and 2.86g of sodium carbonate were added, and the mixture was heated under reflux overnight. The reaction mixture was concentrated *in vacuo*, and a saturated aqueous solution of sodium hydrogen carbonate was added to the residue, followed by extracting the mixture with ethyl acetate. The ethyl acetate phase was washed sequentially with a saturated aqueous solution of sodium hydrogen carbonate and with a saturated brine, and dried over magnesium sulfate. The solvent was evaporated *in vacuo*, and the residue was purified by column chromatography on silica gel (eluent: (1)ethyl acetate, (2)ethyl acetate/methanol=100/6). The oil was azeotropically distilled with toluene to give 7.46 g of (R)-3-[7-cyano-5-[2-[[2-(2-ethoxyphenoxy)ethyl]amino]propyl]-2,3-dihydro-1H-indol-1-yl]propyl benzoate as a brown oil.
¹H-NMR(CDCl₃) δ ppm:
1.04 (d, J=6.0Hz, 3H), 1.41 (t, J=6.9Hz, 3H), 2.10-2.20 (m, 2H), 2.42 (dd, J=13.6, 6.9Hz, 1H), 2.63 (dd, J=13.6, 6.0Hz, 1H), 2.80-3.10 (m, 5H), 3.50-3.60 (m, 2H), 3.75 (t, J=7.3Hz, 2H), 4.00-4.15 (m, 4H), 4.40-4.50 (m, 2H), 6.85-7.00 (m, 6H), 7.40-7.50 (m, 2H), 7.50-7.60 (m, 1H), 8.00-8.10 (m, 2H)
Specific optical rotation: [α]_{D}^{2 7} =-14.8° (c=1.04, methanol)

The resultant compound, (R)-3-[7-cyano-5-[2-[[2-(2-ethoxyphenoxy)ethyl]amino]propyl]-2, 3-dihydro-1H-indol-1-yl]propyl benzoate (7.32g) was dissolved in 46mL of methanol, and then the solution was added to an aqueous solution of 1.54g of potassium hydroxide in 9.2mL of a distilled water, after which the mixture was heated under reflux overnight. The reaction mixture was concentrated *in vacuo*, and 100mL of a distilled water was added to the residue, followed by extracting the mixture with ethyl acetate. The ethyl acetate phase was washed with a saturated aqueous solution of sodium hydrogen carbonate and with a saturated brine, and dried over magnesium sulfate. The solvent was evaporated *in vacuo*, and the residue was dissolved in 30mL of toluene, after which the toluene was evaporated *in vacuo* to give 6.06g of (R)-5-[2-[[2-(2-ethoxyphenoxy)ethyl]amino]propyl]-1-(3-hydroxypropyl)-2, 3-dihydro-1H-indole-7-carbonitrile as a pale brown oil.
¹H-NMR(CDCl₃) δ ppm:
1.05 (d, J=6.0Hz, 3H), 1.41 (t, J=6.9Hz, 3H), 1.50-1.90 (m, 1H), 1.85-2.00 (m, 2H), 2.43 (dd, J=13.6, 6.9Hz, 1H), 2.63 (dd, J=13.6, 6.3Hz, 1H), 2.80-3.10 (m, 5H), 3.50-3.60 (m, 2H), 3.67 (t, J=7.3Hz, 2H), 3.75-3.85 (m, 2H), 4.00-4.15 (m, 4H), 6.85-7.30 (m, 6H)
Specific optical rotation: [α]_{D}²⁷=-19.4° (c=1.06, methanol)

The resultant compound, (R)-5-[2-[[2-(2-ethoxyphenoxy)ethyl]-amino]propyl]-1-(3-hydroxypropyl)-2, 3-dihydro-1H-indole-7-carbonitrile (5.95g) was dissolved in 16.4mL of dimethylsulfoxide, and 0.25mL of 5mol/L aqueous sodium hydroxide was added to the solution. To the reaction mixture, 1.55mL of 30% hydrogen peroxide was added with keeping the reaction temperature 25 ° C or less, and then the mixture was stirred at a reaction temperature of 25 to 30 ° C overnight. To the reaction mixture, 82mL of a solution of 2.39g of sodium sulfite was added, then the mixture was extracted with ethyl acetate. The ethyl acetate phase was washed sequentially with a saturated aqueous solution of sodium hydrogen carbonate, with a distilled water and with a saturated brine, and dried over magnesium sulfate. The solvent was evaporated *in vacuo*, and the residue was recrystallized from ethyl acetate to give 4.72g of (R)-5-[2-[[2-(2-ethoxyphenoxy)ethyl]amino]propyl]-1-(3-hydroxypropyl)-2, 3-dihydro-1H-indole-7-carboxamide.
¹H-NMR(CDCl₃) δ ppm:
1.07 (d, J=6.2Hz, 3H), 1.37 (t, J=7.0Hz, 3H), 1.60-1.85 (m, 3H), 2.54 (dd, J= 13.6, 6.5Hz, 1H), 2.68 (dd, J= 13.6, 6.4Hz, 1H), 2.85-3.10 (m, 6H), 3.19 (t, J=6.6Hz, 2H), 3.35-3.45 (m, 2H), 3.75 (t, J=5.4Hz, 2H), 3.95-4.20 (m, 4H), 5.70(br s, 1H), 6.66(br s, 1H), 6.80-6.95 (m, 4H), 7.02(s, 1H), 7.16(s, 1H)
Specific optical rotation: [α]_{D}^{2 7} =- 15.3° (c=0.98, methanol)

To a solution of 10.9g of the resultant compound, (R)-5-[2-[[2-(2-ethoxyphenoxy)ethyl]amino]propyl]-1-(3-hydroxypropyl)-2, 3-dihydro-1H-indole-7-carboxamide as dissolved in 100mL of methylene chloride, a solution of 5.87g of di-*tert*-butyl dicarbonate in 25mL of methylene chloride was added dropwise under ice-cooling with stirring, and then the mixture was stirred for about 30 minutes under the same conditions, followed by further being stirred for 10 hours at room temperature. The reaction mixture was concentrated *in vacuo*, and the residue was dissolved in 150mL of ethyl acetate, after which the solution was washed sequentially with a 10% aqueous citric acid, with a saturated aqueous solution of sodium hydrogen carbonate and with a saturated brine, and then dried over magnesium sulfate. The solvent was evaporated *in vacuo* to give 10.2g of (R)-N-[2-[7-carbamoyl-1-(3-hydroxypropyl)-2, 3-dihydro-1H-indol-5-yl]-1-methylethyl]-N-[2-(2-ethoxyphenoxy)ethyl]carbamate *tert*-butyl as a pale brown amorphous material
¹H-NMR(CDCl₃ ) δ ppm:
1.20-1.50 (m, 15H), 1.70-1.85 (m, 2H), 2.50-4.40 (m, 18H), 5.75(br s, 1H), 6.63(br s, 1H), 6.80-7.20 (m, 6H)
Specific optical rotation: [α]_{D}^{2 7} =-50.4° (c=1.27, methanol)

To a solution of 6.24g of the resultant compound, (R)-N-[2-[7-carbamoyl-1-(3-hydroxypropyl)-2, 3-dihydro-1H-indol-5-yl]-1-methylethyl]-N-[2-(2-ethoxyphenoxy)ethyl] carbamate *tert*-butyl as dissolved in 9.4mL of dry pyridine, 1.54mL of pivalate chloride was added, and the mixture was heated at room temperature overnight. A saturated aqueous solution of sodium hydrogen carbonate was added to the reaction mixture, and the mixture was extracted with ethyl acetate. The ethyl acetate phase was washed with a saturated aqueous solution of sodium hydrogen carbonate and with a saturated brine, and dried over magnesium sulfate. The solvent was evaporated *in vacuo*, and the residue was purified by column chromatography on aminopropylated silica gel (eluent: hexane/ ethyl acetate=1/1) to give 4.30g of (R)-3-[5-[2-[N- (*tert*-butoxycarbonyl)-N-[2-(2-ethoxyphenoxy)ethyl]amino]propyl]-7-carbamoyl-2, 3-dihydro-1H-indol-1-yl]propyl pivalate as a colorless amorphous material.
¹H-NMR(CDCl₃) δ ppm:
1.15-1.50 (m, 24H), 1.85-2.00 (m, 2H), 2.55-3.20 (m, 6H), 3.30-3.60 (m, 4H), 3.85-4.40 (m, 7H), 5.52(br s, 1H), 6.80-7.40 (m, 7H)
Specific optical rotation: [α]_{D}^{2 7} =-38.3° (c=1.03, methanol)

To a solution of 8.53g of the resultant compound, (R)-3-[5-[2-[N- (*tert*-butoxycarbonyl)-N-[2-(2-ethoxyphenoxy)ethyl]amino]propyl]-7-carbamoyl-2, 3-dihydro-1H-indol-1-yl]propyl pivalate as dissolved in 280mL of methanol, 853mg of 10% palladium carbon and 3.97g of ammonium formate were added, and the mixture was heated for 13 hours under reflux. After the catalysts were removed by filtration, the solvent was evaporated *in vacuo* to give 8.20g of (R)-3-[5-[2-[N- (*tert*-butoxycarbonyl)-N-[2-(2-ethoxyphenoxy)ethyl]amino]propyl]-7-carbamoyl-1H-indol-1-yl]propyl pivalate as a pale green amorphous material.
¹H-NMR(CDCl₃) δ ppm:
1.05-1.50 (m, 24H), 1.90-2.10 (m, 2H), 2.70-3.05 (m, 2H), 3.30-3.75 (m, 2H), 3.85-4.70 (m, 9H), 5.66(br s, 1H), 6.35-6.50 (m, 2H), 6.75-7.55 (m, 7H)
Specific optical rotation: [α]_{D}^{2 7} =-44.5° (c=1.06, methanol)

To a solution of 7.81 g of the resultant compound, (R)-3-[5-[2-[N- (*tert*-butoxycarbonyl)-N-[2-(2-ethoxyphenoxy)ethyl]amino]propyl]-7-carbamoyl-1H-indol-1-yl]propyl pivalate as dissolved in 78mL of isopropanol, 39mL of concentrated hydrochloric acid was added dropwise over 10 minutes under ice-cooling with stirring, and the mixture was stirred for 4 hours at room temperature. After powders of sodium hydrogen carbonate were added to the reaction mixture under ice-cooling with stirring until the pH reached 8, the mixture was diluted with 200mL of water, and the dilution was extracted with ethyl acetate. The ethyl acetate phase was washed sequentially with a saturated aqueous solution of sodium hydrogen carbonate, with water, and with a saturated brine, and then dried over magnesium sulfate. The solvent was evaporated *in vacuo*, and the residue was purified by column chromatography on aminopropylated silica gel (eluent: ethyl acetate), after which the resultant material was recrystallized from diethyl ether/hexane =2/1 to give 5.21g of (R)-3-[7-carbamoyl-5-[2-[[2-(2-ethoxyphenoxy)ethyl]amino]propyl]-1H-indol-1-yl]propyl pivalate as a clear and colorless crystal.
¹H-NMR(CDCl₃) δ ppm:
1.11 (d, J=6.2Hz, 3H), 1.21( s , 9H), 1.27 (t, J=7.0Hz, 3H), 1.95-2.10 (m, 2H), 2.75 (dd, J=13.6, 6.4Hz, 1H), 2.85 (dd, J=13.6, 6.6Hz, 1H), 2.95-3.10 (m, 3H), 3.85-4.00 (m, 4H), 4.00-4.20 (m, 2H), 4.35-4.45 (m, 2H), 5.55-5.65(br s, 1H), 6.05-6.20(br s, 1H), 6.47 (d, J=3.2Hz, 1H), 6.75-6.95 (m, 4H), 7.06 (d, J=3.2Hz, 1H), 7.21 (d, J=1.5Hz, 1H), 7.54 (d, J=1.5Hz, 1H)
Specific optical rotation: [α]_{D}^{2 7} =- 15.8° (c= 1.06, methanol)

### Reference Example 2

### Preparation of (R)-3-[7-carbamoyl-5-[2-[[2-(2-ethoxyphenoxy)ethyl]-amino]propyl]-1H-indol-1-yl]propyl pivalate hydrochloride (KRG-3332)

To a solution of 6.07g of (R)-3-[7-carbamoyl-5-[2-[[2-(2-ethoxyphenoxy)ethyl]amino]propyl]-1H-indol-1-yl]propyl pivalate as obtained in Reference Example 1 in 58mL of ethanol, 11.6mL of 1mol/L hydrochloric acid was added dropwise under ice-cooling with stirring, and the mixture was stirred for 15 minutes under the same conditions. After the reaction mixture was concentrated *in vacuo*, ethanol was added to the residue, and the water was removed by azeotropic distillation. The residue was dissolved in 6ml of ethanol, and 60mL of ethyl acetate was added to the solution, after which the mixture was left for 16 hours at room temperature to give 5.14g of clear and colorless crude crystals. Crude crystals (8.12g) that were obtained by combining the crystals with the other lot of crude crystals that were obtained in a similar manner were recrystallized from ethanol/ ethyl acetate=15/1 to give 7.46g of (R)-3-[7-carbamoyl-5-[2-[[2-(2-ethoxyphenoxy)ethyl]amino]propyl]-1H-indol-1-yl]propyl pivalate hydrochloride as a clear and colorless crystal.
¹H-NMR(CDCl₃ ) δ ppm:
1.21( s , 9H), 1.29 (t, J=7.0Hz, 3H), 1.45 (d, J=6.5Hz, 3H), 1.95-2.10 (m, 2H), 3.12 (dd, J=14.0, 7.2Hz, 1H), 3.30-3.60 (m, 3H), 3.85-4.05 (m, 5H), 4.30-4.50 (m, 4H), 5.87( s , 1H), 6.40 (d, J=3.2Hz, 1H), 6.80-7.00 (m, 4H), 7.05 (d, J=3.2Hz, 1H), 7.33 (d, J=1.5Hz, 1H), 7.36( s , 1H), 7.50 (d, J=1.5Hz, 1H), 9.10-9.30(br s, 1H), 9.50-9.65(br s, 1H)
Specific optical rotation: [α]_{D}^{2 8} =-7.0° (c=1.22, methanol)

### Reference Example 3

### Preparation of (R)-5-[2-[[2-(2-ethoxyphenoxy)ethyl]amino]propyl]-1-(3-hydroxypropyl)-1H-indole-7-carboxamide.

To a solution of 4.93g of (R)-N-[2-[7-carbamoyl-1-(3-hydroxypropyl)-2, 3-dihydro-1H-indol-5-yl]-1-methylethyl]-N-[2-(2-ethoxyphenoxy)ethyl] carbamate *tert*-butyl as prepared in Reference Example 1 as dissolved in 150ml of methanol, 490mg of 10% palladium carbon and 2.96g of ammonium formate were added, and the mixture was heated for 36 hours under reflux. After cooling, the insoluble materials were removed by filtration, the solvent was evaporated *in vacuo*. The residue was dissolved in 150mL of methanol, and 490mg of 10% palladium carbon and 2.96g of ammonium formate were added to the solution, after which the mixture was heated for 24 hours under reflux. The insoluble materials were removed by filtration, and the solvent was evaporated *in vacuo*. The residue was dissolved in ethyl acetate, and the solution was washed with waster and with a saturated brine, and dried over magnesium sulfate. The solvent was evaporated *in vacuo* to give 4.55g of (R)-N-[2-[7-carbamoyl-1-(3-hydroxypropyl)-1H-indol-5-yl]-1-methylethyl]-N-[2-(2-ethoxyphenoxy)ethyl] carbamate *tert*-butyl as a white amorphous material.
¹H-NMR(CDCl₃) δ ppm:
1.05-1.50 (m, 15H), 1.90-2.10 (m, 2H), 2.70-3.00 (m, 3H), 3.30-3.75 (m, 4H), 3.80-4.65 (m, 7H), 5.75-5.95 (m, 1H), 6.40-6.65 (m, 2H), 6.75-7.55 (m, 7H)
Specific optical rotation: [α]_{D}^{3 0} =-47.8° (c=1.05, methanol)

To a solution of 4.45g of the resultant compound, (R)-N-[2-[7-carbamoyl-1-(3-hydroxypropyl)-1H-indol-5-yl]-1-methylethyl]-N-[2-(2-ethoxyphenoxy)ethyl] carbamate *tert*-butyl in 50mL of isopropanol, 25mL of concentrated hydrochloric acid was added in small portions, and then the mixture was stirred for 3 hours at room temperature. Under ice-cooling, a saturated aqueous solution of sodium hydrogen carbonate was added to the reaction mixture, and the mixture was extracted with ethyl acetate. The ethyl acetate phase was washed with a saturated aqueous solution of sodium hydrogen carbonate, and dried over magnesium sulfate. The solvent was evaporated *in vacuo*, and the residue was purified by column chromatography on aminopropylated silica gel (eluent: methylene chloride/ methanol=20/ 1) to give 1.27g of (R)-5-[2-[[2-(2-ethoxyphenoxy)ethyl]amino]propyl]-1-(3-hydroxypropyl)-1H-indole-7-carboxamide as a white amorphous material. Additionally, the mixture that had not been separated was purified by column chromatography on aminopropylated silica gel (eluent: ethyl acetate/ethanol=7/ 1), and the resultant compound was combined with the compound previously purified to give 2.39g of (R)-5-[2-[[2-(2-ethoxyphenoxy)ethyl]amino]propyl]-1-(3-hydroxypropyl)-1H-indole-7-carboxamide as a white amorphous material.
¹H-NMR(CDCl₃) δ ppm:
1.11 (d, J=6.3Hz, 3H), 1.25 (t, J=7.0Hz, 3H), 1.95-2.10 (m, 2H), 2.70-3.20 (m, 6H), 3.52 (t, J=5.6Hz, 2H), 3.93( q , J=7.0Hz, 2H), 4.00-4.20 (m, 2H), 4.38 (t, J=7.0Hz, 2H), 5.90(br s, 1H), 6.38(br s, 1H), 6.49 (d, J=3.2Hz, 1H), 6.75-6.95 (m, 4H), 7.11 (d, J=3.2Hz, 1H), 7.19 (d, J=1.5Hz, 1H), 7.53 (d, J=1.4Hz, 1H)
Specific optical rotation: [α]_{D}^{3 0} =-15.5° (c=1.02, methanol)

### Reference Example 4

### Preparation of (R)-5-[2-[[2-(2-ethoxyphenoxy)ethyl]amino]propyl]-1-(3-hydroxypropyl)-1H-indole-7-carboxamide hydrochloride(KRG-3333)

(R)-5-[2-[[2-(2-Ethoxyphenoxy)ethyl]amino]propyl]-1-(3-hydroxypropyl)-1H-indole-7-carboxamide as prepared in Reference Example 3 (862mg) was dissolved in 5mL of ethanol, and 985µL of 2mol/L hydrochloric acid was added to the solution, after which the solvent was evaporated *in vacuo*. The reside was dissolved in 3 mL of ethanol, and 12mL of ethyl acetate was added to the solution. The mixture was left, and then filtered to remove the precipitated crystals, thereby obtaining 821mg of (R)-5-[2-[[2-(2-ethoxyphenoxy)ethyl]amino]-propyl]-1-(3-hydroxypropyl)-1H-indole-7-carboxamide hydrochloride.
¹H-NMR (dMSO-d₆) δ ppm:
1.19 (d, J=6.4Hz, 3H), 1.26 (t, J=7.0Hz, 3H), 1.70-1.85 (m, 2H), 2.65-2.80 (m, 1H), 3.20-3.55 (m, 5H), 3.64(br s, 1H), 4.02(q, J=7.0Hz, 2H), 4.20-4.40 (m, 4H), 4.55 (t, J=5.0Hz, 1H), 6.45 (d, J=3.1Hz, 1H), 6.85-7.15 (m, 5H), 7.36 (d, J=3.1Hz, 1H), 7.49 (d, J=1.3Hz, 1H), 7.60(br s, 1H), 7.99(br s, 1H), 9.05-9.30 (m, 2H)
Specific optical rotation: [α]_{D}³⁰ =-7.8° (c=1.16, methanol)

### Example 1

### Effects of candidates on expression level of α_{1A}-AR in CHO cells expressing variant α_{1A}-AR

CHO cells that express a receptor of human variant α_{1A}-AR comprising 466 amino acids wherein alanine at 271 position located in the third intracellular loop of human α_{1A}-AR was substituted with threonine were cultured with bunazosin hydrochloride (inverse agonist), KGR-3333 (neutral antagonist), or a mixture thereof, and the expression level of α_{1A}-AR was used to examine the effects of the substances on the α_{1A}-AR activity.

According to British Journal of Pharmacology, Vol.131, pp.546-552 (2000), bovine α_{1A}-AR gene (333bp) (as prepared in reference to J. Biol. Chem., Vol.265, pp. 8183-8189 (1990)) was used as probe to screen human prostate cDNA library (Clontech Inc.), thus isolating the human α_{1A}-AR cDNA fragment having an entire length of 1.5kbp (also comprising the 5' untranslated region of 7bp and 3' untranslated region of ≦ 100bp )

For the resultant human α_{1A}-AR cDNA fragment, alanine at 271 position of the human wild α_{1A}-AR was substituted with threonine by modified site-specific PCR to prepare human variant α_{1A}-AR gene (the receptor gene comprising 466 amino acids wherein alanine at 271 position located in the third intracellular loop of human α_{1A}-AR was substituted with threonine).

The wild or variant α_{1A}-AR gene was incorporated into mammalian expression vector pCR3 (Invitrogen Inc.) using restriction enzyme EcoRI, and the vector was transfected into CHO cells with Lipofectamin (GIBCO Inc.). The transfectants were cultured at 37°C in αMEM (10% fetal bovine serum, 100 units/mL penicillin G, and 100µg/mL streptomycin sulfate) in the presence of 500µg/mL G-418 so as to give the cells that consistently expressed wild or variant α_{1A}-AR.

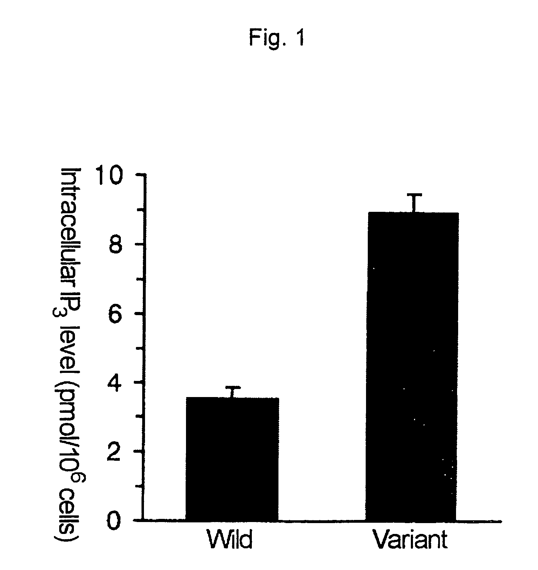

The CHO cells that expressed variant α_{1A}-AR as prepared as shown above were treated with 0.8M perchloric acid, left on the ice for 30 minutes, neutralized with 4M sodium hydroxide containing 60mM EDTA, and centrifuged, and the sediments were removed. The resultant cell extract was examined for intracellular level of IP₃ using inositol-1, 4, 5-trisphosphate [³ H] radioactive receptor assay kit (NEN Inc.). The results are shown in Figure 1. Figure 1 shows that the cells expressing the variant increased in intracellular level of IP₃ compared with the wild, confirming that the active receptor should be predominant in the variant α_{1A}-AR.

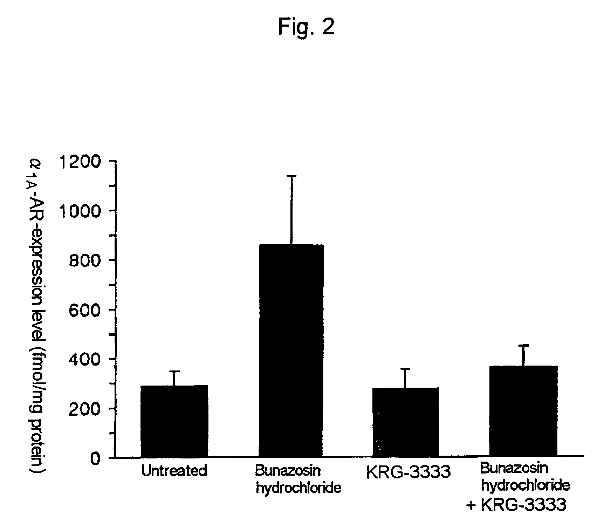

On the other hand, the CHO cells that expressed the variant α_{1A}-AR were cultured at 37°C for 48 hours in the presence of test drugs (bunazosin hydrochloride as prepared according to the publication described above, or KGR-3333 as prepared in Reference Example 4 (10^{- 8} M), or a mixture thereof (each 10^{- 8} M)). Then, the cells were harvested in the isotonic buffer (tris-HCl 50mM, NaCl 125mM, EDTA 2mM, pH7.4), disrupted by sonication, and centrifuged at 80000 x g for 30 minutes, and the resultant sediments were suspended in the assay buffer (tris-HCl 50mM, EDTA 1mM, pH7.4) to prepare membrane fractions. The membrane fractions (about 20 µg protein/tube) were incubated in the presence of [³ H]-prazosin (NEN Inc.) (30 to 2000pM) at 30°C for 45 minutes, and then harvested on GF/C filter (Whatman Inc.) using a cell collecting apparatus (m-36T, Brandel Inc.). The filter was washed with 50mM Tris-HCl buffer (pH7.4) several times, and then the radioactivity bound on the membrane was determined using a liquid scintillation counter. Nonspecific bound was estimated as that in the presence of 1µM tamsulosin hydrochloride (Japanese Patent Publication (kokoku) No. 52742/1987). The protein contents in the membrane fractions of the cells as used were determined with Coomassie (trade mark) plus-200 protein assay reagent (PIERCE Inc.) using bovine serum albumin as standard in accordance with Bradford Method (Anal.Biochem., Vol.pp.248-254(1976)). The experimental data was analyzed using nonlinear approximate program PRISM (trade mark) (Graphpad Software Inc.) to calculate the expression level of the receptor. The results are shown in Figure 2.

Figure 2 shows that, in the experiment using the variant α_{1A}-AR that have been demonstrated predominant in active receptor, the treatment with bunazosin hydrochloride induced the increase in the receptor levels about three times compared to the treatment with KRG-3333, suggesting that bunazosin hydrochloride should be an inverse agonist of α_{1A}-AR. On the other hand, the treatment with KRG-3333 induced no increase in the receptor levels, suggesting that KRG-3333 should be a neutral antagonist. The treatment concomitantly with KRG-3333 and bunazosin hydrochloride almost conserved the receptor levels, suggesting that neutral antagonists inhibited the activity of inverse agonists to increase the receptor levels. In other words, it demonstrated that inverse agonists prevent side effects by neutral antagonists.

### Example 2

### Comparison in development of resistance to activity to reduce intraocular pressure between neutral antagonists and inverse agonists

### (1) Activity to reduce intraocular pressure of 0.01% eye drop of KRG-3332

KRG-3332 as prepared in Reference Example 2 was dissolved in physiological saline to prepare a 0.01% solution, which was used as test solution 1 (0.01% eye drop) in the following method for determining the activity to reduce intraocular pressure.

For the six Dutch male colored rabbits (17 to 19 weeks old, conformed for two weeks or more), intraocular pressure was determined with no eye drop two and one week before the initiation of administration of the test solution to determine that they did not have any aberration in intraocular pressure value and diurnal variation thereof, and then the animals were randomly divided into a group of five for usage in the test, and a group of one for preparative.

For the administration of eye drop, each 50 µL of the test solution and the control (physiological saline) was administered to one eye and the other eye respectively, twice a day (11:00 and 19:00), and the administration continued for up to 57 days. The corneal surfaces of the animals were anaesthetized with 0.4% oxybuprocaine hydrochloride eye drop to , and the intraocular pressure was determined at time points of 11:00 (before the administration), 13:00(2 hours after the administration), 15:00 (4 hours after the administration) and 19:00 (8 hours after the administration, immediately before the second administration) one day before the administration and thereafter every week using an air applanation ophthalmotonometer (ALCON JAPAN LTD.). Average (mean ± S.E.) of the difference between the intraocular pressures of eyes administered with the test solution and the control as determined at each time point, and the relevant t-test results were estimated. The results are shown in Table 1.

**Table 1**

| Test solution 1 (KRG-3332, 0.01% eye drop) Difference in intraocular pressure after the administration (mmHg) | | | | |
|---|---|---|---|---|
| Days | Elapsed time after the administration (hours) | | | |
| | 0 | 2 | 4 | 8 |
| 1st day | 0.050 ± 0.398 | -5.900 ** ± 0.485 | -2.950 * ± 0.788 | -0.400 ± 0.392 |
| 8th day | -0.200 ± 0.242 | -5.850 ** ± 0.674 | -2.550 ** ± 0.200 | -0.800 * ± 0.255 |
| 15th day | -0.950 * ± 0.215 | -4.550 ** ± 0.659 | -1.600 ± 0.882 | -0.350 ± 0.828 |
| 22nd day | -0.850 ± 0.589 | -4.300 ** ± 0.457 | -2.750 ** ± 0.326 | -0.550 ± 0.348 |
| 29th day | -1.500 * ± 0.454 | -5.250 ** ± 1.132 | -2.450 * ± 0.599 | -0.400 ± 0.392 |
| 36th day | -0.500 ± 0.447 | -2.950 ** ± 0.609 | -1.349 ± 0.831 | -1.250 ± 0.818 |
| 43rd day | -0.150 ± 0.491 | -3.900 * ± 0.875 | -2.350 * ± 0.705 | 0.100 ± 0.376 |
| 50th day | -0.400 ± 0.430 | -4.250 * ± 1.135 | -2.300 * ± 0.673 | -0.900 ± 0.551 |
| 57th day | 0.050 ± 0.366 | -4.200 * ± 1.144 | -2.500 ** ± 0.487 | -0.150 ± 0.400 |
| In the Table, "*" means that p value of the relevant t-test is below 0.05, and "**" means that p value is below 0.01. | | | | |

On the all determination days from the initiation to the 57th day, the maximum reduction in intraocular pressure was observed 2 hours after the administration, and there was significant difference compared to the eye administered with the control. Further, the eyes administered with the test solution also showed a significant reduction in intraocular pressure 4 hours after the administration except for the 15th and 36th days.

### (2) Activity to reduce intraocular pressure of 0.1% eye drop of KRG-3332

KRG-3332 was dissolved in physiological saline to prepare a 0.1% solution, which was used as test solution 2 (0.1% eye drop) in the determination of the activity to reduce intraocular pressure in a similar manner to above (1).

The results are shown in Table 2.

**Table 2**

| Test solution 2 (KRG-3332 0.1% eye drop) Difference in intraocular pressure after the administration (mmHg) | | | | |
|---|---|---|---|---|
| Days | Elapsed time after the administration (hours) | | | |
| | 0 | 2 | 4 | 8 |
| 1 st day | 0.000 ± 0.285 | -6.450 ** ± 0.521 | -5.300 ** ± 0.649 | -3.450 ** ± 0.709 |
| 8th day | -1.050 ± 0.619 | -7.050 ** ± 0.644 | -4.200 ** ± 0.483 | -4.000 * ± 1.115 |
| 15th day | -1.350 ± 0.944 | -5.700 ** ± 0.639 | -4.900 ** ± 0.534 | -1.700 ± 0.917 |
| 22nd day | -1.550 * ± 0.414 | -6.800 ** ± 0.752 | -4.800 ** ± 0.713 | -3.550 ** ± 0.704 |
| 29th day | -1.350 ** ± 0.232 | -5.800 ** ± 0.310 | -3.900 ** ± 0.669 | -3.200 * ± 0.885 |
| 36th day | -1.050 ± 0.878 | -5.150 ** ± 0.683 | -3.950 ** ± 0.686 | -2.950 * ± 0.992 |
| 43rd day | -1.800 * ± 0.515 | -4.000 ** ± 0.622 | -2.900 ** ± 0.302 | -2.250 ± 0.862 |
| 50th day | -0.600 ± 0.801 | -3.900 ** ± 0.777 | -1.950 ± 0.756 | -1.150 ± 0.573 |
| 57th day | -1.500 ± 0.822 | -3.850 ** ± 0.625 | -2.650 ** ± 0.478 | -1.700 ** ± 0.348 |
| In the Table, "*" and "**" are the same meaning as defined in Table 1 of above (1). | | | | |

Table 2 shows that, on the all determination days from the initiation to the 57th day, the maximum reduction in intraocular pressure was observed 2 hours after the administration similarly to above (1), and also shows that there was significant difference compared to the eye administered with the control. Further, the eyes administered with the test solution also showed a significant reduction in intraocular pressure 4 hours after the administration except for the 50th day.

### (3) Activity to reduce intraocular pressure of 0.03% eye drop of bunazosin hydrochloride

For the comparison, bunazosin hydrochloride was dissolved in physiological saline to prepare a 0.03% solution, which was used as test solution 3 (reference drug, 0.03% eye drop of bunazosin hydrochloride) in the determination of the activity to reduce intraocular pressure in a similar manner to above (1) and (2).

The results are shown in Table 3.

**Table 3**

| Test solution 3 (0.03% eye drop of bunazosin hydrochloride) Difference in intraocular pressure after the administration (mmHg) | | | | |
|---|---|---|---|---|
| Days | Elapsed time after the administration (hours) | | | |
| | 0 | 2 | 4 | 8 |
| 1 st day | 0.700 ± 0.320 | -4.400 ** ± 0.809 | -1.700 ± 1.452 | 0.150 ± 0.947 |
| 8th day | 0.600 ± 0.579 | -3.100 ** ± 0.465 | -0.950 ± 1.176 | -0.350 ± 0.430 |
| 15th day | 0.100 ± 0.562 | -1.450 ± 0.804 | -0.250 ± 0.494 | -0.100 ± 0.150 |
| 22nd day | -0.050 ± 0.567 | -0.250 ± 0.576 | -0.200 ± 0.330 | 0.350 ± 0.150 |
| 29th day | 0.300 ± 1.011 | -0.250 ± 0.652 | -0.500 ± 0.306 | -0.200 ± 0.289 |
| In the Table, "*" and "**" are the same meaning as defined in Table 1 of above (1) and (2). | | | | |

Table 3 shows that, in a group of 0.03% eye drop of bunazosin hydrochloride, although the eyes administered with the test solution showed a significant reduction in intraocular pressure 2 hours after the administration on 1st and 8th days from the initiation, the degree of the reduction was smaller on the 15th day, and any difference from the eyes administered with the control was hardly observed on the 22nd day. On the 29th day, a similar result was observed, and therefore the test for administration with 0.03% eye drop of bunazosin hydrochloride was discontinued.

### (4) Results

As described above, Dutch male colored rabbits were used to examine the relationship between development of drug resistance and inverse agonist activity of KRG-3332 that is one of prodrugs of a neutral antagonist of α_{1A}-AR KRG-3333, and of bunazosin hydrochloride that is an inverse agonist of α_{1A}-AR. 0.01% and 0.1% eye drops of KRG-3332, and 0.03% eye drop of bunazosin hydrochloride were administered to the eyes of test animals for up to 57 days (8 weeks), and the intraocular pressure was determined once a week, so that the change in their activity to reduce intraocular pressure as induced by the continuous administration was observed. The tests revealed that the bunazosin hydrochloride-treatment group showed that the degree of the reduction in intraocular pressure was smaller on the 15th day from the initiation (2 weeks after the initiation), and any reduction in intraocular pressure was hardly observed on the 22nd day (3 weeks after the initiation), whereas the KRG-3332-treatment group showed a significant reduction in intraocular pressure even on the 57th day (8 weeks after the initiation), which indicated no development of drug resistance.

### (5) Discussion

The test results as described above show that α_{1A}-AR neutral antagonists that exhibit no inverse agonist activity in CHO cells expressing variant α_{1A}-AR are extremely effective for treatment or prevention of ocular hypertensions, since such α_{1A}-AR neutral antagonists cause no attenuation of activity to reduce intraocular pressure, and therefore obviate drug resistance, and rebound phenomenon that deteriorates the condition after the discontinuation.

### Formulation 1

### Eye ointment

KRG-3332 as prepared in Reference Example 2 is taken in a portion of 0.1g, and the portion is levigated with 5.0g of liquid paraffin to give a slurry. White petrolatum is added to the slurry in small portions until the total weight is 100.0g, which is then filled into a tight container.

### Formulation 2

### Eye drop

KRG-3332 as prepared in Reference Example 2 is taken in a portion of 0.1g, and the portion is dissolved in an appropriate amount of purified water, after which 0.6g of acetic acid, 0.4g of sodium chloride and 0.005g of benzalkonium chloride are added to the solution. An appropriate amount of 1 mol/L sodium hydroxide is added to the solution to adjust the pH to 5.0, and then purified water is added until the total amount is 100 mL. The solution is sterilized by filtration, and filled aseptically into a sterilized container for eye drop.

### Formulation 3

### Eye drop

KRG-3332 as prepared in Reference Example 2 (0.1g) and bunazosin hydrochloride (0.1g) are dissolved in an appropriate amount of purified water, and 2.1g of citric acid, 0.4g of sodium chloride and 0.005g of benzalkonium chloride are added to the solution. An appropriate amount of 1 mol/L sodium hydroxide is added to the solution to adjust the pH to 6.0, and then purified water is added until the total amount is 100 mL. The solution is sterilized by filtration, and filled aseptically into a sterilized container for eye drop.

### INDUSTRIAL APPLICABILITY

As described above, pharmaceutical compositions for treatment and prevention of a glaucoma or an ocular hypertension, and for administration to various patients thereof, which exhibit a strong activity to reduce intraocular pressure, and which induce no increase in the number of α_{1A}-AR can be prepared by incorporating neutral antagonists as effective ingredients into the compositions.

## Claims

1. An ophthalmic pharmaceutical composition which comprises an α_{1A}-adrenergic receptor neutral antagonist as an effective ingredient.

2. The pharmaceutical composition according to claim 1, wherein the α_{1A}-adrenergic receptor neutral antagonist is a compound of formula: 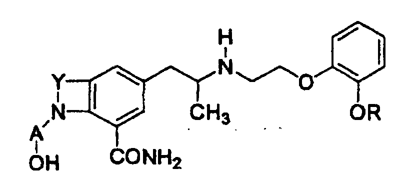 in which A is a lower alkylene, R is a lower alkyl or a halo(lower alkyl), and Y is ethylene or vinylene; a prodrug thereof, and a pharmaceutically acceptable salt of them.

3. The pharmaceutical composition according to claim 1, wherein the α_{1A}-adrenergic receptor neutral antagonist is selected from the group consisting of (R)-5-[2-[[2-(2-ethoxyphenoxy)ethyl]amino]-propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]-propyl]-1H-indole-7-carboxamide, a prodrug thereof, and a pharmaceutically acceptable salt of them.

4. The pharmaceutical composition according to claim 3, wherein the prodrug is (R)-3-[7-carbamoyl-5-[2-[[2-(2-ethoxyphenoxy)-ethyl]amino]propyl]-1H-indol-1-yl]propyl pivalate, or a pharmaceutically acceptable salt thereof.

5. The pharmaceutical composition according to any one of claims 1 to 4, which is used together with an α_{1A}-adrenergic receptor inverse agonist.

6. The pharmaceutical composition according to claim 5, which comprises further an α_{1A}-adrenergic receptor inverse agonist.

7. The pharmaceutical composition according to any one of claims 1 to 6, which is used for treatment or prevention of a glaucoma or an ocular hypertension.

8. A pharmaceutical composition as used for α_{1A}-adrenergic receptor neutral antagonist, which comprises at least one selected from the group consisting of (R)-5-[2-[[2-(2-ethoxyphenoxy)ethyl]amino]-propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]-propyl]-1H-indole-7-carboxamide, a prodrug thereof, and a pharmaceutically acceptable salt of them.

9. The pharmaceutical composition as used for α_{1A}-adrenergic receptor neutral antagonist according to claim 8, which comprises (R)-3-[7-carbamoyl-5-[2-[[2-(2-ethoxyphenoxy)ethyl]amino]-propyl]-1H-indol-1-yl]propyl pivalate, or a pharmaceutically acceptable salt thereof.

10. A pharmaceutical composition comprising an α_{1A}-adrenergic receptor neutral antagonist, which is for prevention of a side effect by an α_{1A}-adrenergic receptor inverse agonist.

11. The pharmaceutical composition according to claim 10, which comprises further an α_{1A}-adrenergic receptor inverse agonist.

12. A method for treating or preventing a glaucoma or an ocular hypertension, which comprises administering a therapeutically effective amount of an α_{1A}-adrenergic receptor neutral antagonist to a patient in need.

13. The method according to claim 12, wherein the α_{1A}-adrenergic receptor neutral antagonist is selected from the group consisting of a compound of formula: 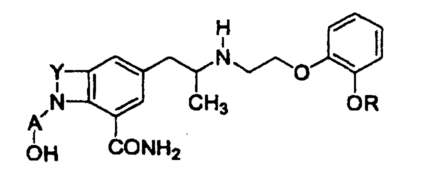 in which A is a lower alkylene, R is a lower alkyl or a halo(lower alkyl), and Y is ethylene or vinylene; preferably (R)-5-[2-[[2-(2-ethoxyphenoxy)-ethyl]amino]propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]-amino]propyl]-1H-indole-7-carboxamide, and a prodrug thereof, more preferably (R)-3-[7-carbamoyl-5-[2-[[2-(2-ethoxyphenoxy)ethyl]amino]-propyl]-1H-indol-1-yl]propyl pivalate, and a pharmaceutically acceptable salt thereof.

14. The method according to claim 12 or 13, which comprises administering a therapeutically effective amount of an α_{1A}-adrenergic receptor neutral antagonist to a patient who are receiving an α_{1A}-adrenergic receptor inverse agonist.

15. A method for preventing a side effect occurred in a patient who is receiving an α_{1A}-adrenergic receptor inverse agonist, which comprises administering a therapeutically effective amount of an α_{1A}-adrenergic receptor neutral antagonist to the patient.

16. Use of an α_{1A}-adrenergic receptor neutral antagonist for the manufacture of a pharmaceutical composition for treatment or prevention of a glaucoma or an ocular hypertension.

17. The use according to claim 16, wherein the α_{1A}-adrenergic receptor neutral antagonist is selected from the group consisting of a compound of formula: 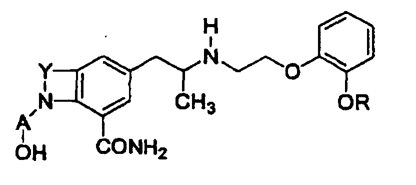 in which A is a lower alkylene, R is a lower alkyl or a halo(lower alkyl), and Y is ethylene or vinylene; preferably (R)-5-[2-[[2-(2-ethoxyphenoxy)ethyl]amino]propyl]-1-(3-hydroxypropyl)-1H-indole-7-carboxamide, (R)-1-(3-hydroxypropyl)-5-[2-[[2-[2-(2,2,2-trifluoroethoxy)phenoxy]ethyl]amino]propyl]-1H-indole-7-carboxamide, and a prodrug thereof, more preferably (R)-3-[7-carbamoyl-5-[2-[[2-(2-ethoxyphenoxy)ethyl]amino]propyl]-1H-indol-1-yl]propyl pivalate, and a pharmaceutically acceptable salt thereof.

18. Use of an α_{1A}-adrenergic receptor neutral antagonist and an α_{1A}-adrenergic receptor inverse agonist, for the manufacture of a pharmaceutical composition for treatment or prevention of a glaucoma or an ocular hypertension.

19. The use according to claim 18, wherein the α_{1A}-adrenergic receptor neutral antagonist is selected from a compound of formula as defined in claim 17, and a pharmaceutically acceptable salt thereof.
